# Europäisches Patentamt

## European Patent Office

## Office européen des brevets

(11) Publication number: **0 077 430**
**B1**

# EUROPEAN PATENT SPECIFICATION

(45) Date of publication of patent specification: **02.01.86**

(51) Int. Cl.⁴: **A 61 K 7/06, A 61 K 9/48**

(21) Application number: **81830197.0**

(22) Date of filing: **16.10.81**

(54) A pharmaceutical composition for enhancing keratinogenesis and reducing facial and scalp seborrhea.

(43) Date of publication of application:
27.04.83 Bulletin 83/17

(45) Publication of the grant of the patent:
02.01.86 Bulletin 86/01

(84) Designated Contracting States:
AT BE CH DE FR GB LI LU NL

(56) References cited:
DE-A-2 331 456
FR-A-2 228 470

CHEMICAL ABSTRACTS, vol. 85, no. 7, 16th
August 1976, page 426, column 1, no. 45314b,
Columbus Ohio (USA); J. SCALA et al.: "Effect
of daily gelatine ingestion on human scalp
hair".

The file contains technical information
submitted after the application was filed and
not included in this specification

(73) Proprietor: **Mavi S.r.l.**
Via Filippo Bernardini 22
I-00165 Roma (IT)

(72) Inventor: **Morganti, Pier Francesco**
Via Montoggio 49
I-00168 Roma (IT)

(74) Representative: **Bazzichelli, Alfredo**
c/o Società Italiana Brevetti Piazza Poli 42
I-00187 Roma (IT)

(56) References cited:
CHEMICAL ABSTRACTS, vol. 73, no. 19, 9th
November 1970, page 131, column 1, no.
95985u, Columbus Ohio (USA); V.V. KALININ
et al.: "Effectiveness of synthetic DL-
methionine in the ration of sheep".
Journal of Applied Cosmetology, Vol. (2) No. 1,
Jan-March 1984

Courier Press, Leamington Spa, England.

**Description**

This invention refers to a medicinal preparation having the object of promoting keratinogenesis, that is of reinforcing the formation of hair and nails as well as reducing seborrhoea of the skin of the face and of the scalp.

Gelatin (denominated officially in the official Pharmacopoeia "F. U. Gelatin" or according to the international denomination "Gelatinum") is a known substance which is used, particularly, for the production of medicinal capsules.

The use is also known, especially in the pharmaceutical industry, of capsules made of gelatin so-called "soft" which are used as containers of various pharmaceutical active principles. Such capsules, known in fact as soft gelatin capsules, are produced with a particular process of fusion completely automatic and continuous known as the Scherer method ("rotary die process") as described, for example, in U.S. patents 2318718, 2356436, 2333433, 2379816, 2379817, 2451141, or through other methodologies.

Such process involves melting of the gelatin with glycerin, sorbitol and other substances suitable for improving its physical properties, in order to obtain as final product a unity of "soft" gelatin as is well known in the art. During or after melting, other active principles (proteins, amino acids, mineral salts, vitamins, etc.) which are thermally stable at 70°C, may be added to the gelatin.

It is also known that it is possible to produce noticeable modifications in proteins having a high sulphur content; enrichening the diet of sheep with cysteine or with sulphurated amino acids. To this end see Reis P. J. and Schinckel P. G. (1963) Aust. J. Biol, Sci. 16, 218; Reis P. J. (1965) Aust. J. Biol. Sci. 18, 671; Gillespie J. M. Broad A. and Reis P. J. (1969) Biochem. J. 112, 41; Broad A., Gillespie J. M. and Reis P. J. (1970) Aust. J. Biol. Sci. 23, 149; Frenkel M., Gillespie J. M. and Reis P. J. (1974) Aust. J. Biol. Sci. 27, 31.

Also by adding collagen gelatins to the diet it is possible to see a further increase of the keratinic fraction having a high sulphur content (Gillespie J. M., Reis J. P. and Schinckel P. G. (1964) Aust. J. Biol. Sci. 17, 532.

It is equally known that the oral consumption of gelatin increases significantly the degree of hardness of nails, thus solving problems of nail fragility (see for example Thyson T. T. (1950) J. Invest. Dermatol, 14, 323).

The surprising effect which gelatin has been found to have on keratinogenesis seems to be attributable to a "specific dynamic action" due to the high percentage of glycine it contains (see Michaelson J. B. and Huntsman D. (1963) J. Soc. Cosmet. Chem. 14, 443.

The observation also seems to have a certain importance, that the protein chains of the gelatin are formed by amino acids present in the same proportions and arranged in a similar way with respect to corresponding amino acids of the keratins, with the only exception of cystin.

In the description and claims according to the invention, the term "cystine" is exclusively intended to refer to the levo form of cystine, with the exclusion of the optically isomeric dextro-cystine.

On the other hand Bigwood E. J. and Robazza F. (1955) have found in the hair of African children affected by Kwashiorkor, a level of cystine below that of other healthy children which live in the same area (see Bigwood E. J. and Robazza F. (1955) Voeding 87, 251).

It has been later observed that hyponutrition delays the rhythm of growth, the diameter and resistance to tension of the hair of pigs the diet of which had been modified in order to obtain marasmatic and Kwashiorkorlike conditions (see Cabak V., Gresham G. A. and McCance R. A. (1962) Brit. J. Nitr. 16, 635).

Finally, in the recent years it has been clearly shown that the relative type and proportions of the proteins forming the corneous keratins play a decisive role in determining certain mechanical properties. In fact, whils the principal factor in the stabilization of the keratinic structures of hair and human nails is represented by the disulfide bridges and therefore by the presence of proteins having a high content in cystine, proteins rich in glycine and tyrosine should not be neglected which, although present in human hair in small quantities, seem to contribute in a significant way in the stability and resistance of the keratinic structures of several animals (see Bendit E. G. and Gillespie J. M. (1978) Biopolymers 17, 2743).

The sebonormalizing action is also known as the elective stimulating action carried out by the cystine in keratinization processes of the hairy structures, which therefore result the hair is more resistant the higher its cystine content is. In fact, due to its sebo regulating action and due to its action which controls the keratinization of the skin and nails and stimulates the growth of hair, cystine is used in the treatment of various stages of alopecia, in seborrhoic conditions in general, in the onicodistrophies and in nail and hair fragility in doses from 1 to 2 g a day.

In Chemical Abstract 85:45314b (1976) A. J. Scala et al., Nutr. Rep. Int. 1976, 13(6), pages 579—592, there is mentioned a favourable effect of daily gelatine ingestion on human scalp hair. DE—A—2331456 teaches that 1-cystine has a favourable effect on hair and suggests the use of capsules for the administration of this agent.

However, the two last mentioned references do not teach a combination of gelatin and cystine as an agent promoting hair growth and reducing seborrhoea. DE—A—2331456 shows a combination of six main ingredients among which cystine is included and no indication of the role cystine should play in the combination is given. Moreover, though no mention is made of the daily dosage of the combination to be administered, it can be assumed that, since each formulation contains only 1 mg of cystine, the effect of administering cystine is negligeable with the effect resulting from our application which provides an administration of 800 mg cystine a day, an amount which is greater by a factor of hundreds than that suggested by the cited prior art.

Moreover the combination of gelatin/cystine resulting from the mere fact that cystine could be contained in gelatin capsules, would lead to a "mechanical mixture" of gelatin and cystine which is specifically excluded from the scope of the present invention.

It has now been discovered according to the invention that solid spherules of gelatin produced according to the Scherer method and therefore formed by gelatin and glycerin or sorbitol, to which is added a certain quantity of cystine, orally administered in daily doses comprising about 800 mg of cystine in 2 g of gelatin, have an improving and stimulating effect on keratinogenesis and at the same time reduces the seborrhoea both of the face and of the scalp.

According to the invention this effect is due to the association of "soft" gelatin with cystine, offering results which are superior to those yielded by presently available formulations which contain only gelatin or only cystine, or which contain both in different proportions.

Object of the present invention is a medicinal preparation for promoting the keratinogenesis in hair and reducing the seborrhoea of the face and of the scalp for oral administration, in the form of solid and homogeneous spherules of soft gelatin comprising 65 to 80% by weight of Gelatinum and 25 to 30% by weight of glycerin or sorbitol, characterized in that said spherules contain as an agent active for promoting keratinogenesis and reducing seborrhoea 20 to 25% by weight of levo-cystine with respect to the total weight of the preparation, homogeneously dispersed in said soft gelatin.

In fact, today formulations do not exist on the market which are formed by so-called "soft" gelatin which contain also cystine and it will be noticed that the doses indicated by the available literature in order to obtain the desired effects are much higher (3 to 6 g of gelatin per day or 1 to 2 g per day of cystine), with respect to the dose according to the formulation of the present invention which contains only 2 g of soft gelatin and 800 mg of cystin to be administered daily.

The fact should be particularly pointed out that cystine which forms the activating agent is not contained in the gelatin in the usual way in which the active principles are contained in normal gelatin capsules, but the levo-cystine is distributed in a homogeneous way in a solid spherule, with no cavity, in the indicated proportions.

According to the invention in fact the intimate distribution of the cystine within the mass of "soft" gelatin ensures a particularly favorable oral absorption, so that it is possible to reduce the doses of active substances with respect to the doses established by the prior art for the indicated pharmaceutical use. To the end of demonstrating the synergical effect of the ingestion of cystine and gelatin through administration of soft gelatin capsules, experiments have been carried out on rats in which the condition of loss of hair was induced by means of a biotin free diet. It is in fact known that the alimentary insufficiency of biotin induces, in the rat, the formation of a seborrhoic clinical condition, characterized by the increase of the sebaceous secretion and by the fall of hair.

In order to have a direct comparison, said rats have been successively treated, in different lots, with diets to which was added respectively gelatin, cystine, gelatin-cystine in mechanical mixture and gelatin-cystine in soft spherules.

Experimental test 1
Methods and materials
60 Whistar rats were employed of both sexes (30 males and 30 females) of the average weight of 150 g±9. Following the Kalopissis et al methodology (U.S. patent No. 3629452) 50 rats were kept on a biotin free diet for about 40 days until an evident seborrhoic clinical condition had developed, while the remaining 10 served as controls. After obtaining the desired clinical condition with the development of seborrhoea and loss of hair in various zones, the 50 rats were subdivided into five groups of 10 rats each and treated as follows:

1st group:  biotin free diet added with cystine;
2nd group:  biotin free diet added with gelatin;
3rd group:  biotin free diet added with cystine and gelatin in mechanical mixture;
4th group:  biotin free diet added with the mixture of gelatin/cystine melted in soft spherules;
5th group:  biotin free diet;
6th group:  control.

The diets were administered weekly in the cages of the rats in accurately weighed quantity so that the rats, kept in single cages, would ingest together with the feed, respectively:

1st group:  84 mg per kg a week of cystine;
2nd group:  203 mg per kg a week of gelatin from collagen;
3rd group:  84 mg per kg a week of cystine and 203 mg per kg a week of gelatin;
4th group:  280 mg per kg a week of the melted mixture of gelatin/cystine comprising 79.8 mg of cystine and 200.2 mg of gelatin.

3

# 0 077 430

After three weeks of treatment and after taking about 200 mg of hairs per rat, all the animals, included the controls, were sacrificed in a chloroform saturated environment in order to control their skin total lipids and in order to carry out possible histological and bioptic examinations.

Determination of the sulphur content of the hair

The hair of all the animals (about 100 mg), thoroughly washed with alcohol and ethyl ether, and dried, was used for the determination of the total sulphur carried out by titration according to the McDonald methodology (McDonald A. M. G. (1959) Industr. Chem. 35, 33). The results expressed as sulphur percentage with respect to the dried weight of the hair, are given in Table 1.

Determination of the total lipids

Immediately after sacrificing the animals, dorsal samples of skin were taken, which, freed from the paniculus adiposus, were used both for the determination of the total lipids as well as for the histochemical studies.

The content of the total lipids was determined gravimetrically by the difference in weight of the sample before and after the extraction in a Soxhlet with acetone for 10 days and further drying out in vacuum over $P_2O_5$ and paraffin for 24 hours. The values, expressed in percentage, are listed in Table 1.

TABLE 1

Effects on the skin and hair of rats kept on a biotin poor diet

| Group | Number of speci- mens | Biotin poor diet | Additive | Total lipides % | Number of determi- nations | Sulphur content (×g hair, %) | Number of determi- nations |
|---|---|---|---|---|---|---|---|
| I | 10 | Yes | Levo-cystine | 38.1±2.987° | 30 | 2.48±0.066° | 20 |
| II | 10 | Yes | Gelatin | 43.5±3.005° | 30 | 2.44±0.08° | 20 |
| III | 10 | Yes | Levo-cystine°° and gelatin | 35.8±2.796° | 30 | 2.74±0.062° | 20 |
| IV | 10 | Yes | Levo-cystine* and gelatin | 26.9±3.111° | 30 | 3.19±0.075° | 20 |
| V | 10 | Yes | — | 46.2±3.102° | 30 | 2.32±0.067 | 20 |
| VI | 10 | No | — | 29.7±3.015 | 30 | 3.90±0.081 | 20 |

°=P<0.05 versus group VI (controls).
°°=mechanical mixture.
*=mixture obtained by melting according to the Scherer process.

Comments and results

As it can be seen from Table 1, in the rats kept on a biotin poor diet it is possible to notice a reduction in the sulphur in the hair of about 15% and a global increase of the total lipids of the skin of about 50%. If furthermore the data are observed relating to the individual treatments, it may be clearly seen that the oral assumption of the cystine alone (12 mg/kg/die) causes, in the biotin-poor rats, an increase of the total sulphur of the hair of about 7% (P<0.05) and a reduction of the total lipids of the skin of about 15% (P<0.05). The oral assumption of gelatin alone, on the contrary, while raising the sulphur content of the hair by about 5% (P<0.05) does not cause any reduction of the lipidic condition of the skin of the biotin-poor rats.

Furthermore the combined action of the cystine and gelatin, which, added to the diet, demonstrated a synergistic action of stimulating both the keratinogenesis as well as the lipogenesis, appears also to be very interesting. In fact both the increase found in the level of the total sulphur in the hair and the reduction of the lipids in the skin in rats fed a biotin poor diet with levo-cystine and gelatin, appears to be greater than the summation of the action obtained singularly both with the levo-cystine and the gelatin. This synergistic action increases noticeably if the simple mechanical mixture is substituted with the same mixture obtained by melting through the Scherer process. In fact, at equal concentration both of cystine and of gelatin, almost a doubling of the action demonstrated both at the keratinogenesis and lipogenesis level is observed.

Experimental test 2

The elective stimulating action has also been verified on rats, performed especially by the gelatin

4

cystine, in the keratinization processes of the hairy structures, verifying the average content of sulphur and cystine of the hair of rats kept on a normal diet and respectively of rats kept on a biotin poor diet, fed respectively only gelatin, only cystine and the association of gelatin-cystine both in the form of mechanical mixture in hard capsules, and in the form melted in soft spherules.

Materials and methods

110 Whistar rats of both sexes were employed (55 males plus 55 females) of the average weight of 150 g±9, divided in two lots of respectively 60 (lot I) and 50 (lot II) rats each. Following the Kalopissis and Manoussos methodology already quoted in test 1, 50 rats of lot I were kept on a biotin poor diet for about 40 days until the appearance of an evident seborrhoic clinical condition was induced, while the remaining 10 served as controls. After having obtained the desired clinical condition with the appearance of seborrhoea and loss of hair in several zones, the 60 rats were divided into six groups of 10 rats each and treated as follows:

1st group: normal laboratory diet (controls);
2nd group: biotin free diet;
3rd group: biotin free diet with cystine added;
4th group: biotin free diet with gelatin added;
5th group: biotin free diet with cystin gelatin in mechanical mixture (hard capsules) added;
6th group: biotin free diet with melted mixture of gelatin-cystine (soft spherules) added.

The diets prepared specifically by a specialized firm were administered weekly in the cages of the rats, in accurately weighed quantity so that the rats, kept in single cages, would ingest together with the feed, respectively:

1st group: normal diet;
2nd group: biotin (BC) poor diet;
3rd group: diet BC+84 mg per kg weekly of cystine;
4th group: diet BC+203 mg per kg weekly of gelatin FU;
5th group: diet BC+84 mg per kg weekly of cystine and 203 mg per kg weekly of gelatin FU (hard capsules);
6th group: diet BC+280 mg per kg weekly of the melted mixture of gelatin-cystine composed of 79.8 mg of cystine and of 200.2 mg of gelatin (soft spherules).

After six weeks of treatment and after having taken about 1 g of hair per rat, all the animals included the controls were sacrificed in a chloroform saturated atmosphere.

The 50 rats of the second lot (normal rats, not subjected previously to a biotin poor diet), divided into five groups of 10 rats each, have been fed respectively:

1st group: normal laboratory diet (DN) (controls);
2nd group: DN+cystine;
3rd group: DN+gelatin FU
4th group: DN+gelatin and cystine in mechanical mixture (hard capsules);
5th group: DN+gelatin and cystine previously melted together (soft spherules).

The quantity of the feed placed in the cages was calculated in such a way that the rats would ingest the same quantity per kg of cystin, FU gelatin or gelatin-cystine already described above.

After 150 days of treatment the rats, under ether anaesthesia, were partially depilated with a razor.

Determination of the sulphur content of the hair

A part of the hair (about 100 mg) of all the animals of the two groups, thoroughly washed with alcohol and ethyl ether and dried, are used for the determination of the total sulphur carried out by means of titration, according to the previously mentioned McDonald methodology. The results expressed as percentage of sulphur with respect to the dried weight of the hairs, are listed in Tables 2 and 3.

Determination of the cystine content in the hair

After having hydrolyzed the hair of all the animals (about 800 mg) previously washed as already described, the quantity of cystine was determined using an autoanalyzer.

The results expressed as g/100 g of hairs are listed in Tables 2 and 3.

Determination and isolation of the proteins in the hair

The extraction of the total keratins of the hairs was carried out using the urea-thioglycolate method as described in Gillespie J. M., Reis P. J. and Schinckel P. G. (1964) Aust. J. Biol. Sci. *17*, 548.

After determining from one portion the total keratin (total proteins), the keratin fraction of high sulphur content is separated through alkylation with iodo acetate, dialysis and precipitation of the protein of low

5

sulphur content with zinc acetate at pH 6. The total sulphur was determined on the protein fraction "rich in sulphur" according to the already mentioned McDonald methodology. The results are listed in Tables 2 and 3.

TABLE 2

Variations in the composition of hair keratin of biotin poor rats fed on a gelatin-cystine enriched diet

| Treatment | Sulphur content % | Cystine content % | Total proteins % | "Sulphur rich" proteic fraction % | Sulphur contained in the "sulphur rich" proteic fraction, % |
|---|---|---|---|---|---|
| Normal diet (control) | 3.90±0.081° | 12.0±1 | 90±1 | 24±2 | 5.9±0.2 |
| Biotin poor diet (BC) | 2.32±0.07° | 8.0±0.5 | 91±2 | 17±1 | 3.3±0.03 |
| BC/levo-cystine | 2.48±0.07° | 9.3±0.6 | 90±1 | 20±1 | 3.9±0.05 |
| BC/gelatin | 2.44±0.08° | 9.1±0.8 | 90±1 | 18±0.5 | 3.6±0.04 |
| BC/levo-cystine/ gelatin | 2.74±0.06° | 10.3±0.5 | 91±2 | 27±1 | 4.3±0.05 |
| BC/levo-cystine/ gelatin | 3.19±0.07° | 12.0±0.8 | 90±1 | 28±1 | 4.9±0.08 |

°P=0.05 versus normal diet.

TABLE 3

Variations in the composition of keratin of the hair of rats fed on a gelatin-cystin enriched diet

| Treatment | Sulphur content % | Cystine content % | "Sulphur rich" keratin % | Sulphur contained in the "sulphur rich keratin" % |
|---|---|---|---|---|
| Normal diet (DN) | 3.3±0.1° | 11.8±0.5° | 21.0±0.5° | 4.7±0.1° |
| DN/levo-cystine (84 mg/kg weekly) | 3.6±0.1° | 13.8±0.6° | 25.9±0.5° | 5.1±0.2° |
| DN/gelatin (203 mg/kg weekly) | 3.1±0.1° | 11.5±0.5° | 23.9±0.6° | 4.5±0.1° |
| DN/gelatin/cystine (203+84 mg/kg weekly), hard capsules | 3.8±0.2° | 14.3±0.5° | 31.7±0.5° | 5.5±0.2° |
| DN/gelatin/cystine (280 mg/kg weekly), soft spherules | 4.2±0.2° | 15.5±0.6° | 32.5±0.6° | 6.1±0.2° |

°P=0.05 versus normal diet.

Comments and results

From Table 2 significant differences can be seen between the group of rats on the biotin poor diet and the control group (P<0.05) at all levels. In fact, the lack of biotin causes in the rat a reduction of the total sulphur present in the hair by about 50%. Such reduction, considering the analytical difficulties, seems to be totally due to a corresponding decrease in the content of cystine (about 40% P<0.05).

It is interesting to notice furthermore that the reduction of both the sulphur and of the cystine is accompanied by a noticeable reduction (about 50%) of the protein fraction so-called "rich in sulphur" present essentially, as known, in the matrix of the hair. As the rats are gradually fed the diet enriched with cystine and the mixture of gelatin-cystine, a significant protein increase noticeable at the level of the protein fraction of the matrix of the hair is noticed. The protein increase is accompanied always by a corresponding increment in cystine.

Observing the data concerning the rats lacking biotin fed respectively gelatin, cystine and cystine-gelatin both in the form of hard capsules and of soft spherules it is proven that the alimentary enrichment with gelatin alone, in the dosages employed, while it does not seem to be able of bringing significant improvements in the global mass of parameters taken into consideration, if administered together with cystine, it boosts its activity in a substantial way.

In fact, while the addition to the diet of cystine alone increases the parameters taken into consideration, the alimentary enrichment with gelatin-cystine seems to be able practically to bring the values related to the matrix keratins (fraction "rich in sulphur") and of the cystine of the hair itself back to normal. The gelatin-cystine administered in the pharmaceutical form of soft spherules (premelting of the gelatin-cystine) seems to be able also to further increase the incorporation of the cystine at the level of the matrix of the hair, may be due to the different bioavailability proven by such pharmaceutical form.

From the data concerning normal rats fed for a long period (20 weeks) with diets enriched with gelatin-cystine and mixture of the two (Table 3) a progressive increase is immediately seen in the sulphur, in the cystine and in the keratin fraction "rich in sulphur" of the matrix of the hair. An increment is obtained in fact of the keratin fraction "rich in sulphur" which ranges from 10% for the rats fed with only gelatin to 50% for the rats fed with the association of gelatin and cystine administered in the form of soft spherules. It seems therefore clear that the increase in the cystine which can be found in the hair of rats fed with a diet enriched with gelatin-cystine is due especially to the increase of the proteins of the matrix of the hair. The gelatin, probably due to its high content in glycine, seems to be capable of increasing noticeably the incorporation of the cystine in the keratin fraction (rich in sulphur) of the matrix of the hair. Such increase appears to be greater, furthermore depending on the pharmaceutical form used probably due to a different bioavailability of the feed itself. In fact, as it can be seen from Table 2, the major content of cystine has been found in the hair of rats fed with the enriched diet with the association of gelatin and cystine in the form of soft spherules (premelted mixture). This diet has caused also the major increment both of the protein fraction "rich in sulphur" (+50%) as well of the cystine which can be found in the hair (+33%) of the rats treated with respect to the controls (P<0.05).

Experimental test 3

Experimental test on humans

To the end of verifying the possible stimulating selective action caused by cystine and gelatin in the keratinizing process of the hairy structures, a study has been conducted in order to control the possible beneficial effect deriving from enrichment of the human diet with gelatin and cystine in well defined proportions.

Materials and methods

40 voluntary Italian people having dark hair and suffering from a Effluvium capillitii of both sexes and of ages between 20 and 40 were selected. All voluntary patients received, through the double blind technique, a bottle containing spherules of gelatin-cystine (product A) of spherules of placebo (product B). Before beginning the experiment and 24 hours after the last time the hair had been washed, the number of the follicular orifices (sites) and the number of hairs (fibers) emerging from each site, carrying out the count in a well defined area (1 cm×1 cm) between the rear vertex (VP) and the front vertex (VA) were checked, with an illuminated magnifying glass, according to the Moretti subdivision (Moretti G., Baccaredda-Boy A. and Rebora A. (1969) in "W. Montagna and R. L. Dobson" "Advances in Biology of the Skin", Vol. IX, "Hair Grow", Pergamon Press, Oxford, page 538).

Methodology of the count

The count of the sites and fibers was carried out using a small transparent perspex ruler 10 cm in length with a center opening 1 cm$^2$ wide, according to the Cottington et al (J. Soc. Cosmet. Chem. 28, 219) methodology, during a period of 3 months. Furthermore, before beginning the treatment, about 200 mg of hair in the area near the counting point was sampled from all the voluntary patients, cutting the sample just barely above the scalp with well sharpened scissors. During the treatment period the voluntary patients have always used the same shampoo and also avoided cutting the hair.

Determination of sulphur and free thiols

After having calculated the mean thickness according to the Cottington methodology already mentioned, about 100 mg of the sample hair, previously cleansed with alcohol and ethyl ether, are used to determine the quantity of total sulphur according to the already mentioned McDonald methodology. On the remaining integral portion (about 100 mg) the total number of free thiols is determined amperometrically.

During the three months of treatment, the voluntary patients took during normal meals four spherules a day of the alimentary supplement, equal, for the A product, of 2 g of gelatin and 800 mg of cystine a day, and for the B product, to about 100 mg of gelatin and 300 mg of starch.

The results are listed in Table 4.

TABLE 4

Effects of the hair of gelatin-cystine after a three months treatment

| Treatment | Medium fibers per cm² | Medium sites per cm² | Medium fibers per sites | Increase % | Medium diameter μm | Increase % | Sulphur content (×g hair) % | Increase % | Medium content free thiols μm×g hair | Reduction % |
|---|---|---|---|---|---|---|---|---|---|---|
| Gelatin/ cystine | before 155.3±48° | 84.2±15* | 1.84±0.49 | | 61.5±5.4 | | 3.82±0.08 | | 251±30 | |
| | | | | 49.4° | | 35° | | 26.7° | | 62.9° |
| | after 270.6±37° | 98.5±17* | 2.75±0.21 | | 83.2±8.1 | | 4.84±0.10 | | 93±15 | |
| Placebo | before 167.5±33* | 79.3±16* | 2.11±0.63* | | 67.3±8* | | 3.91±0.11* | | 192±25* | |
| | | | | — | | — | | — | | — |
| | after 174.0±39* | 82.9±20* | 2.09±0.37* | | 61.0±10* | | 3.74±0.12* | | 173±19* | |

°=P0.05
°°=P0.005
*=insignificant

Results and comments

As it can be seen from Table 4, the average increase of fibers/sites is equul to about 50% with P<0.05. This means that the average number of the hairs emerging from the fullicular orifices themselves (sites) are doubled within 3 months in those individuals subjected to a diet enriched with gelatin-cystine. As a matter of fact those patients fed with the placebo (product B) underwent no improvement.

Similar results have emerged also with regard both to the total sulphur content, which increases by about 27% (P<0.05), and of the free thiols present on the integral hair, for which a decrease is seen of over 60% (P<0.005).

The experimental test demonstrates that the preparation according to the invention may be employed to cure hair or seborrhoea in humans.

According to the invention it was found that very satisfactory results are obtained with spherules produced through the Scherer method having the following composition of percentage by weight:

| | |
|---|---|
| gelatin | 50—60% |
| glycerin or sorbitol | 18—23% |
| cystine | 20—25% |

The following composition is particularly preferred:

| | |
|---|---|
| gelatin | 57% |
| glycerin | 20% |
| cystine | 23% |

The "soft" gelatin per se has the following composition in percentage by weight:

| | |
|---|---|
| gelatin | 55—80% |
| glycerin or sorbitol | 23—30% |

The preferred composition being:

| | |
|---|---|
| gelatin | 73.5% |
| glycerin | 26.5% |

It should be particularly noted that the cystine constitutes the activating agent is not contained in the gelatin in the usual way in which the active principles are contained in normal capsules of gelatin, but the cystine is distributed in a homogeneous way in a solid spherule, having no cavities, in the indicated proportions.

According to the invention, in fact the intimate distribution of the cystine in the mass of "soft" gelatin ensures a particularly favorable oral absorption.

Preferably each spherule will have a total weight of about 900 mg, in which the active substances are subdivided in the following way:

| | |
|---|---|
| gelatin | 450 to 550 mg |
| glycerin or sorbitol | 160 to 200 mg |
| cystine | 180 to 220 mg |

In a particularly preferred embodiment, each spherule contains:

| | |
|---|---|
| gelatin | 500 mg |
| glycerin or sorbitol | 180 mg |
| cystine | 200 mg |

The daily oral dose is considered to be 4 spherules a day, so that the total daily dosage appears to be about 2 g of gelatin and 800 mg of cystine.

It is further obvious that the spherules of gelatin may contain other pharmaceutically compatible additives, such as edulcorants, dyes, vitamins, mineral elements, and natural extracts, as long as they are thermally stable at 70°C.

The gelatin spherules may be produced through melting of the gelatin itself with glycerin, sorbitol or other substances suited to improve the physical properties according to the "Scherer" method or through similar methodologies which yield as a final product homogeneous units of "soft" gelatin of any whatsoever form and color.

**Claims**

1. Medicinal preparation for promoting the keratinogenesis in hair and reducing the seborrhoea of the face and of the scalp, for oral administration in the form of solid and homogeneous spherules of soft gelatin comprising 65 to 80% by weight of Gelatinum and 25 to 30% by weight of glycerin or sorbitol, characterized in that said spherules contain as an agent active for promoting keratinogenesis and reducing seborrhoea 20 to 25% by weight of Levo-cystine with respect to the total weight of the preparation, homogeneously dispersed in said soft gelatin.

2. The preparation according to claim 1, wherein each spherule has a total weight of about 880 mg and comprises 23% levo-cystine by weight.

3. The preparation according to claim 1 or 2, further comprising pharmaceutically compatible edulcorant and dyeing additives.

**Patentansprüche**

1. Pharmazeutisches Praeparat zur Foerderung der Haarkeratinogenese und Reduzierung von Seborrhoea der Gesichts- und Kopfhaut in Form fester und homogener Kuegelchen aus Weichgelatine, fuer orale Verabreichung, welche 65 bis 80 Gew.% Gelatinum und 25 bis 30 Gew.% Glyzerin oder Sorbit enthalten, dadurch gekennzeichnet, dass die Kuegelchen als aktiven Wirkstoff zur Foerderung der Keratinogenese und zur Reduzierung der Seborrhoea 20 bis 25 Gew.% Levocystin mit Bezug auf das Gesamtgewicht des Praeparats, homogen in der Weichgelatine dispergiert, enthalten.

2. Praeparat gemaess Anspruch 1, wobei jedes Kuegelchen ein Gesamtgewicht von etwa 880 mg hat und 23 Gew.% Levocystin enthaelt.

3. Praeparat gemaess Anspruch 1 oder 2, welches ausserdem pharmazeutisch vertraegliche Suessmittel und Farbstoffe enthaelt.

**Revendications**

1. Préparation pharmaceutique pour augmenter la kératinogénèse des cheveux et réduire la séborrhée faciale et du cuir chevelu, pour administration orale, sous forme de sphérules pleines et homogènes de gélatine molle comprenant de 65 à 80% en poids de Gelatinum et 25 à 30% en poids de glycérine ou sorbite, caractérisée en ce que les dites sphérules contiennent, comme agent actif pour augmenter la kératinogénèse et réduire la séborrhée, de 20 à 25% en poids de levocystine, par rapport au poids de la préparation, dispersé de manière homogéne dans la dite gélatine molle.

2. Préparation selon la revendication 1, où chaque sphérule a un poids total d'environ 880 mg et contient 23% en poids de levocystine.

3. Préparation selon la revendication 1 ou 2, comprenant en outre des additifs édulcorants et colorants pharmaceutiquement compatibles.